# EUROPEAN PATENT SPECIFICATION

(11) **EP 2 531 222 B1**
(45) Date of publication and mention of the grant of the patent: **02.04.2014**
(21) Application number: 11709472.2
(22) Date of filing: 03.02.2011
(51) Int. Cl.: A61K 47/48, A61K 9/127, A61P 9/00, A61P 17/00, A61P 27/02

(54) **LIPOSOMES CONTAINING PROSTAGLANDIN E1 (PGE1), FORMULATIONS CONTAINING THEM AND THEIR USE**
LIPOSOMEN MIT PROSTAGLANDIN E1 (PGE1), FORMULIERUNGEN DAMIT UND IHRE VERWENDUNG
LIPOSOMES CONTENANT DE LA PROSTAGLANDINE E1 (PGE1), FORMULATIONS LES CONTENANT ET UTILISATION ASSOCIÉE

(30) Priority: 03.02.2010 IT FI20100013
(43) Date of publication of application: 12.12.2012
(73) Proprietor: Bioricerca Di Giovanni Brotzu & C. SNC, 09127 Cagliari (IT); Fase 1 S.r.l., 09123 Cagliari (IT)
(72) Inventor: BROTZU, Giovanni, I-09127 Cagliari (IT)
(74) Representative: Gitto, Serena
(86) International application number: PCT/IB2011/050463
(87) International publication number: WO 2011/095938

(56) References cited:
- TOYOTA T ET AL: "Lipo-PGE1, a new lipid-encapsulated preparation of prostaglandin E1: Placebo-and prostaglandin E1-controlled multicenter trials in patients with diabetic neuropathy and leg ulcers", PROSTAGLANDINS, BUTTERWORTH, STONEHAM, MA, US LNKD- DOI:10.1016/0090-6980(93)90081-H, vol. 46, no. 5, 1 November 1993 (1993-11-01), pages 453-468, XP023446007, ISSN: 0090-6980 [retrieved on 1993-11-01]

## Description

### Field of the invention

The present invention relates to the field of pharmaceutical formulations and particularly those comprising liposomes.

### State of the art

The purpose of the present invention is to make available efficient and safe liposomes encapsulating prostaglandin E1 (PGE1) for the treatment (following systemic administration) of vascular disorders in diabetic patients, and for the local treatment (following topical administration) of skin ulcers and diabetic retinopathies.

When administered systemically, prostaglandin E1 (PGE1) is known to be rapidly metabolised in the lung so the majority of its efficacy is obviously lost. To overcome the above-mentioned problem, liposomes of phosphatidylcholine or other phospholipids have been studied to ascertain the feasibility of obtaining the release of the PGE1-α-cyclodextrin complex for the treatment of pathological conditions associated with diabetes (see, for instance, Toyota T. et al.: Lipo-PGE1. A new lipid-encapsulation of prostaglandin E1: placebo and prostaglandin E1 controlled trails in patients with diabetic neuropathy and leg ulcers. Prostaglandins 1993, 46, 453-468.

In previous research (see Golub M. et al.: Metabolism of prostaglandins A1 and E1. J. Clin. Invest. 1975; 56, 1404-1410), liposomes of phosphatidylcholine were used as the drug carrier to enable the PGE1 to pass beyond the pulmonary filter and avoid it being metabolised by 15-hydroxy dehydrogenase.

Given the importance of the use of PGE1-α-cyclodextrin in treating the vascular diseases of diabetics, and for the local treatment of skin ulcers and diabetic retinopathies, there is an evident need to develop new formulations capable of overcoming the above-mentioned problems, ensuring an effective extension of the half-life of the PGE1 administered and facilitating the drug's release at the required site.

### Summary of the invention

Unilamellar liposomes encapsulating PGE1 and/or PGE1-α-cyclodextrin, in combination with L-propionyl carnitine, the external surface of which is coated with hydrophilic polymers are described.

### Detailed description of the invention

The present invention allows to overcome the abovedescribed problems thanks to the combination of unilamellar liposomes comprising prostaglandin E1 with L-propionyl-carnitine, on the external surface of which hydrophilic polymers are present.

In fact, it has surprisingly been found that said polymer coating not only enables a better adhesion to the cells of the endothelial tissue, but also improves the stability of the liposome in the plasma and consequently prolongs its circulation time with a consequent increase in the drug's half-life and an improved capacity of the delivery system to bind to a specific site. The unilamellar liposomes according to the invention consist of a phospholipid vesicle containing a nucleus of aqueous solution.

The phospholipids constituting the wall of the vesicle are natural or synthetic phospholipids, given their high biocompatibility and non-toxicity.

The phospholipids suitable for use according to the invention include, for instance: phosphatidylcholine (lecithin), phosphatidylethanolamine, phosphatidylserine, phosphatidylglycerol, phosphatidylinositol, dimyristoylphosphatidylcholine (DPMC), dipalmitoylphosphatidylcholine (DPPC), distearoylphosphatidylcholine (DSPC), palmitoyl-stearoylphosphatidylcholine, sphyngomyelin.

The liposomes may also include additives that serve as stabilisers or surface charge modifiers, such as cholesterol, cholesterol sulphate.

The aqueous solution contained in the vesicle consists of phosphate buffer or physiological solution.

The liposomes as described above encapsulate the active ingredient (be it in free form [PGE1] or in the form of an inclusion compound [PGE1-α-cyclodextrin]) and L-proplonyl-carnitine, the purpose of which is to facilitate the beta-oxidation of the lipid chains, increasing the metabolism of the endothelial cells.

The external surface of the liposomes is then coated with hydrophilic polymers, such as poly-L-lysine (PLL), polyornithine and fibronectin, or mixtures thereof.

Poly-L lysine is preferred to ensure adhesion to the cells in culture plates, since it has been demonstrated that poly-L lysine makes the liposome adhere to the endothelial cells.

The small unilamellar vesicles (SUV) according to the invention can be prepared, using known techniques, starting from multilamellar liposome vesicles (MLV) by extrusion of the latter through polycarbonate filters, using a suitable extruder, or by homogenisation.

The SUV thus obtained are then coated with the hydrophilic polymers by "dropping" the liposomes drop by drop into the polymer solution under constant stirring.

The multilamellar liposome vesicles (MLV) can also be prepared, according to known methods, by dispersion of all the components (phospholipids, any stabiliser, PEG1 and/or PGE1-α-cyclodextrin, carnitine) in phosphate buffer (pH 7.4) under mechanical stirring at a temperature higher than the transition temperature (Tc) of the phospholipid being used. The resulting MLV are used to obtain SUV by extrusion through a polycarbonate membrane or by homogenisation. The SUV thus obtained are then purified by elimination of the unencapsulated active ingredient by dialysis.

The formulations for systemic use therefore consist of the liposomes thus obtained suitably diluted - with saline solution, for instance - to obtain a formulation suitable, for example, for systemic administration by venous injection in a slow infusion over 24 hours.

If formulations for topical use (e.g. for the treatment of ulcers or retinopathies, as mentioned previously) are required, the liposomes are dispersed in suitable polymer films.

Said films are known and are prepared using organic polymers, such as sodium hyaluronate, hydroxypropylcellulose (HPMC), polyethylene glycol 400 (PEG 400) and water, in appropriate proportions, and are characterised on the basis of their visco-elastic properties, thickness and bio-adhesion in vitro, using a rheometer, a micrometer and a tensiometer, respectively.

The films are then used to prepare the medication for in situ application consisting, for instance, of strips of various sizes to be applied over lesions or on the surface to be treated. They may also be in the form of contact lenses to apply directly to the eye for the treatment of retinopathies.

If required, the formulations can also be lyophilised and subsequently reconstituted at the time of their use.

The efficiency (E%) of encapsulation of the active ingredient and the carnitine in the liposomes was determined by HPLC (obviously after having broken down the liposomes with a suitable membrane lysing agent, such as Triton X-100.)

The liposomes were characterised on the basis of their dimensional, polydispersion index (PI) and z potential, while their morphological and structural characteristics were studied under transmission electron microscope (TEM) and polarised-light optical microscope.

In vitro release studies were performed using Franz diffusion cells.

The coated and uncoated liposomes were then tested in vitro, using endothelial cells to assess the effect of the polymers used on the vesicle's adhesion to said cells.

The distribution of the PGE1 inside the cells was also evaluated.

The cells were treated with the liposomes coated with hydrophilic polymer and containing PGE1 (in free and complex form) . The liposomes were "marked" with hydrophilic and lipophilic dyes to enable them to be located inside the cell. At the end of the experiment, the cells were fixed and studied under the microscope to ascertain the liposomes' internalisation and their location.

The invention is now further illustrated in the light of the examples given below.

### Example 1

The liposomes were prepared in aseptic conditions using sterile raw materials. A solution of phosphatidylcholine (50mg/ml), PGE1 (free or compound) (60µg/ml) and L-propionyl-carnitine (10mg/ml) was used. The components were placed in a suitable sterile container with sterile aqueous medium (buffer, pH 7.4.)

The dispersion was maintained under constant mechanical stirring for 2 hours. A homogeneous colloidal (vesicular) dispersion of multilamellar liposome (MLV) was thus obtained.

The MLV were then extruded using polycarbonate filters with a pore diameter of 50 nm to obtain small unilamellar vesicles (SUV).

The unilamellar liposomes were then dialysed to remove any unencapsulated drug.

The resulting liposomes were then "poured drop by drop" into a solution of polylysine (MW 150,000-300,000) at a concentration corresponding to (0.01-1 %) and maintained under constant stirring for 2-3 hours to guarantee the electrostatic interaction of the (positively charged) hydrophilic polymer with the (negatively charged) liposomal surface. The interaction between the liposome and the polymer was identified from the variation in the surface charge (the z potential becomes positive) and from the dimensions of the vesicles (from 60 nm to 98 nm).

The resulting liposomes can then be lyophilised and preserved at room temperature, and they can be used for the preparation of formulations for systemic administration, e.g. for dilution in a 0.09% saline solution.

Alternatively, if formulations for topical application need to be prepared, the coated or uncoated liposomes are incorporated in polymer films for the topical administration of the drug. The films are prepared using sodium hyaluronate, hydroxypropylcellulose (HPMC), polyethylene glycol 400 (PEG 400) and water, in appropriate proportions.

The films are characterised in vitro on the basis of their visco-elastic properties, thickness and bio-adhesion, using a rheometer, a micrometer and a tensiometer, respectively. Here again, the in vitro release studies are performed using Franz diffusion cells.

### Liposome characterisation

The mean diameter of the liposomes was found to be 60 nm with a polydispersion index of 0.2.

The quantity of PGE 1 contained in the liposomes after purification was in the range of 30-50 µg/ml, while the quantity of carnitine was in the range of 0.05 to 0.2 mg/ml.

The liposomes were characterised on the basis of their dimensions, polydispersion index (PI) and z potential, respectively, by photon correlation spectroscopy (PCS) (dimensions and PI) and M3-PALS (phase analysis light scattering), which measures the electrophoretic mobility of the particles in a thermostat-controlled cell (z potential), using the Zetasizer Nano (Malvern Instruments, UK).

## Claims

1. Unilamellar liposomes comprising PGE1 in combination with I-carnitine wherein on the external surface of said liposomes hydrofile polymers are present.

2. Unilamellar liposomes according to Claim 1 comprising natural or synthetic phospolipides chosen among: lecithin phosphatidylethanolamine, phosphatidylserine, phosphatidylglycerol phosphatidylinositole, dimyristoylphosphatidylcholine - DPMC, dipalmitoylphosphatidylcoline DPPC, distearoylphosphatidylcholine DSPC, palmitoyl-stearoylphosphatidylcholine sphyngomieline possibly in combination with additives acting as stabiliser or modifier of the supercial charge.

3. Unilamellar liposomes according to Claim 1 wherein said PGE1 is E1 prostaglandine both in free form or as inclusion complex PGE1-a-cyclodextrine.

4. Unilamellar liposomes according to Claim 1 wherein said hydrofile polymers are chosen among: Poly-L-Lisine - PLL, polyornithine and fibronectine or mixtures thereof.

5. Process for the preparation of liposomes according to Claims 1 -4 wherein said liposomes are obtained by homogenisation or extrusion through polycarbonate filters of multi-lamellar liposomes.

6. Formulations for systemic administration comprising liposomes according to Claims 1 - 4.

7. Formulations for topic application containing liposomes according to Claims 1 - 4.

8. Formulations according to Claim 7 wherein said formulations comprise liposomes according to claims 1 - 4 dispersed in polymer films.

9. Formulations according to Claim 8 wherein said polymer films are prepared using organic polymers chosen among: sodium hyaluronate, hydroxypropylcellulose - HPMC, polyethylenglycole400 and water in suitable ratios.

10. Formulations according to Claim 9 consisting of strips to be applied "in situ".

## Patentansprüche

1. Unilamellare Liposomen, umfassend PGE1 in Kombination mit L-Carnithin, wobei auf der äußeren Oberfläche der Liposomen hydrophile Polymere vorliegen.

2. Unilamellare Liposomen nach Anspruch 1, umfassend natürliche oder synthetische Phospholipide, welche aus Lecithin, Phosphatidylethanolamin, Phosphatidylserin, Phosphatidylglycerin, Phosphatidylinosit, Dimyristoylphosphatidylcholin (DMPC), Dipalmitoylphosphatidylcholin (DPPC), Distearoylphosphatidylcholin (DSPC), Palmitoylstearoylphosphatidylcholin, Sphingomyelin ausgewählt sind, gegebenenfalls in Kombination mit Additiven, die als Stabilisator oder Modifikator der Oberflächenladung fungieren.

3. Unilamellare Liposomen nach Anspruch 1, wobei das PGE1 Prostaglandin E1, in freier Form oder als Einschlusskomplex PGE1-α-Cyclodextrin, ist.

4. Unilamellare Liposomen nach Anspruch 1, wobei die hydrophilen Polymere aus Poly-L-Lysin (PLL), Polyornithin und Fibronektin oder Mischungen davon ausgewählt sind.

5. Verfahren zur Herstellung von Liposomen nach den Ansprüchen 1 - 4, wobei die Liposomen mittels Homogenisierung oder Extrusion durch Polycarbonatfilter von multilamellaren Liposomen erhalten werden.

6. Formulierungen zur systemischen Verabreichung, umfassend Liposomen nach den Ansprüchen 1 - 4.

7. Formulierungen zur topischen Applikation, enthaltend Liposomen nach den Ansprüchen 1 - 4.

8. Formulierungen nach Anspruch 7, wobei die Formulierungen Liposomen nach den Ansprüchen 1 - 4, dispergiert in Polymerfilmen, umfassen.

9. Formulierungen nach Anspruch 8, wobei die Polymerfilme unter Verwendung von organischen Polymeren, die aus Natriumhyaluronat, Hydroxypropylcellulose (HPMC), Polyethylenglykol 400 ausgewählt sind, und Wasser in geeigneten Verhältnissen hergestellt worden sind.

10. Formulierungen nach Anspruch 9, bestehend aus Streifen zur Applikation "in situ".

## Revendications

1. Liposomes unilamellaires comprenant de la PGE1 en combinaison avec de la 1-carnitine, dans lesquels sur la surface externe desdits liposomes des polymères hydrophiles sont présents.

2. Liposomes unilamellaires selon la revendication 1 comprenant des phospholipides naturels ou de synthèse choisis parmi : la lécithine, la phosphatidyléthanolamine, la phosphatidylsérine, le phosphatidylglycérol, le phosphatidylinositol, la dimyristoylphosphatidylcholine - DPMC, la dipalmitoylphosphatidylcholine DPPC, la distéaroylphosphatidylcholine DSPC, la palmitoyl-stéaroylphosphatidylcholine, la sphingomyéline éventuellement en combinaison avec des additifs agissant en tant que stabilisant ou modificateur de la charge superficielle.

3. Liposomes unilamellaires selon la revendication 1, dans lesquels ladite PGE1 est une prostaglandine E1 sous une forme non combinée ou sous la forme d'un complexe d'inclusion PGE1-a-cyclodextrine.

4. Liposomes unilamellaires selon la revendication 1, dans lesquels lesdits polymères hydrophiles sont choisis parmi : la Poly-L-Lysine - PLL, la polyornithine et la fibronectine ou un de leurs mélanges.

5. Procédé de préparation de liposomes selon les revendications 1 à 4, dans lequel lesdits liposomes sont obtenus par homogénéisation ou par extrusion à travers des filtres en polycarbonate de liposomes multi-lamellaires.

6. Formulations destinées à l'administration par voie générale, comprenant des liposomes selon les revendications 1 à 4.

7. Formulations destinées à l'application locale, contenant des liposomes selon les revendications 1 à 4.

8. Formulations selon la revendication 7, lesdites formulations comprenant des liposomes selon les revendications 1 à 4 dispersés dans des films de polymères.

9. Formulations selon la revendication 8, dans lesquelles lesdits films de polymères sont préparés en utilisant des polymères organiques choisis parmi : le hyaluronate de sodium, l'hydroxypropylcellulose - HPMC, le polyéthylène glycol 400 et de l'eau en des proportions relatives appropriées.

10. Formulations selon la revendication 9 consistant en des bandes à appliquer *« in situ* ».
